Europäisches Patentamt

European Patent Office

Office européen des brevets

(19)

(11) **EP 0 641 587 B1**

(12) **EUROPÄISCHE PATENTSCHRIFT**

(45) Veröffentlichungstag und Bekanntmachung des Hinweises auf die Patenterteilung:
**14.01.1998 Patentblatt 1998/03**

(51) Int. Cl.$^6$: **B01D 29/01**, B01D 29/58, B01D 21/26

(21) Anmeldenummer: **94112739.1**

(22) Anmeldetag: **16.08.1994**

(54) **Verfahren zum Auftrennen einer Dispersion von Partikeln in Flüssigkeiten in einen mit Partikeln angereicherten und einen an Partikeln verarmten Teilstrom**

Process for splitting dispersions of particles in liquids into a particle-rich and a particle poor stream

Procédé pour séparer des dispersions de particules dans un liquide dans des portions riches et pauvres en particules

(84) Benannte Vertragsstaaten:
**BE CH DE ES FR GB IT LI NL SE**

(30) Priorität: **27.08.1993 DE 4328885**

(43) Veröffentlichungstag der Anmeldung:
**08.03.1995 Patentblatt 1995/10**

(73) Patentinhaber: **BAYER AG**
**51368 Leverkusen (DE)**

(72) Erfinder:
**Koglin, Bernd, Prof. Dr.**
**D-51467 Bergisch Gladbach (DE)**

(56) Entgegenhaltungen:
**DE-U- 8 915 369      GB-A-  930 205**

## Beschreibung

Die Erfindung betrifft ein Verfahren, mit dem kontinuierlich eine strömende Dispersion von Partikeln in einer Flüssigkeit in einen mit Partikeln angereicherten und einen an Partikeln verarmten Teilstrom zerlegt wird.

Verschiedene Vorrichtungen und Verfahren zur Lösung dieser Aufgabenstellung sind bekannt. Hierzu zählen die Sedimentation im Schwerefeld und in Zentrifugen, verschiedene Filtrationsverfahren, die Flotation sowie spezielle Trenntechniken, die beispielsweise elektrische oder magnetische Felder nutzen.

Insbesondere wird auf die folgenden bekannten kontinuierlichen Strömungstrennverfahren hingewiesen.

Im Hydrozyklon erfolgt die Trennung aufgrund der Zentrifugalkraft. Diese wird von einer Wirbelströmung erzeugt, die durch den tangentialen Einstrom der Dispersion in ein Rohr erzeugt wird. Im Zentrum des Rohrs kann die an Partikeln verarmte Flüssigkeit abgezogen werden, während die am Rohrmantel mit Partikeln angereicherte Dispersion durch eine überlagerte axiale Strömung entfernt wird. Aufgrund seines Trennprinzips eignet sich der Hydrozyklon nur für Partikel mit deutlichen Dichtedifferenzen zur Flüssigkeit. Auch bei Dichtedifferenzen von einigen g/cm$^3$ ergeben sich ausreichende Trenneffekte nur für Partikelgrößen oberhalb von etwa 5 μm.

Bei der Querstromfiltration strömt die Dispersion beispielsweise durch ein Rohr mit poröser Wand. Mit Hilfe eines Überdruckes wird gleichzeitig Flüssigkeit durch die poröse Wand, beispielsweise eine Membran gepreßt. Werden die Partikel von der porösen Wand zurückgehalten, so ist die Permeatflüssigkeit klar. Durch die Querströmung wird die Filterkuchenbildung an der Rohrinnenwand verhindert oder begrenzt, so daß mit der Kernströmung eine mit Partikeln angereicherte Dispersion ausgetragen wird. Voraussetzung für die Querstromfiltration ist, daß die Poren der Membran feiner sind als die Partikel der Dispersion. Es sind Membranen mit sehr feinen Poren verfügbar, die praktisch beliebig feine Partikel zurückhalten. Nachteilig ist jedoch, daß mit zunehmender Feinheit der Poren der Durchströmungswiderstand und die Verstopfungsgefahr steil ansteigen. Insbesondere verformbare Partikel verstopfen die Membran und bilden eine dichte, nahezu flüssigkeitsundurchlässige Filterkuchenschicht, die auch durch Querströmung nicht vollständig entfernt werden kann.

Der Erfindung liegt die Aufgabe zugrunde, ein möglichst einfaches Verfahren und eine Vorrichtung zur Aufkonzentrierung von Suspensionen zu entwickeln, das sowohl bei verschwindender Dichtedifferenz der Partikel, wenn Sedimentationsverfahren versagen, als auch bei feinen verformbaren Partikeln, wo Kuchen- oder Querstromfiltrationsverfahren versagen, gute Ergebnisse, d. h. einen hohen Partikelabscheidegrad bei ausreichendem Durchsatz liefert.

Diese Aufgabe wird erfindungsgemäß dadurch gelöst, daß man die aufzukonzentrierende Suspension als Aufgabestrom durch einen engen Kanal oder eine Kapillare mit einer Spaltweite bzw. mit einem Durchmesser von 0,5 μm bis 10 mm, vorzugsweise 10 μm bis 10 mm, strömen läßt und am Umfang des Kanals bzw. der Kapillare durch Öffnungen in der Kanalwand bzw. Kapillarenwand, deren Spaltweite bzw. Durchmesser größer ist als der Medianwert der Partikelgrößenverteilung des Aufgabestroms, Teilströme von der Wand aus abzieht, wobei aufgrund der im Aufgabestrom in Wandnähe abnehmenden Partikelkonzentration im Vergleich zum Aufgabestrom die Partikelkonzentration im Hauptstrom angereichert und in den Teilströmen verdünnt wird.

Die vorliegende Erfindung nutzt einen neuen Effekt, der weder auf dem Prinzip der Zentrifugalkraft noch auf dem Prinzip der Filtration beruht. Der Effekt beruht vielmehr auf der in einer Strömung in Wandnähe abnehmenden Partikelkonzentration. Deshalb wird das erfindungsgemäße Verfahren im folgenden als "Strömungskonzentration" und das erfindungsgemäße Trennorgan als "Strömungskonzentrator" bezeichnet. In besonders günstigen Fällen läßt sich nicht nur eine Aufkonzentrierung, sondern eine vollständige Abscheidung der Partikel erreichen.

Vorzugsweise werden die wandnahen Teilströme mit einer Richtungskomponente entgegen dem Hauptstrom abgezogen, wobei der Umlenkwinkel vorteilhaft zwischen 120° und 150° liegt.

Weiterhin wird das Verfahren zweckmäßig so durchgeführt, daß im Hauptstrom turbulente Strömungsbedingungen und in den Teilströmen laminare Strömungsbedingungen vorherrschen.

Die Strömungsverhältnisse werden gemäß einer bevorzugten Ausführung so eingestellt, daß der Teilmengenstrom aus den Öffnungen in gleicher axialer Höhe 2 % bis 10 %, vorzugsweise 4 % bis 7 % des Hauptmengenstroms beträgt.

Besonders hohe Abscheidegrade kann man erreichen, wenn in Strömungsrichtung gesehen hintereinander kaskadenförmig jeweils auf gleicher axialer Höhe Teilströme abgezogen werden.

Vorzugsweise wird das Verfahren so durchgeführt, daß die Summe aller Teilströme bis zu 99 % des Aufgabestromes beträgt.

Das Verfahren kann auch vorteilhaft zur Klassierung von Partikeln verschiedener Größe verwendet werden. Zu diesem Zweck werden die aus verschiedenen axialen Höhen abgezogenen Teilströme getrennt abgeführt, um Teilströme mit unterschiedlicher Partikelkonzentration und unterschiedlicher Größenverteilung der Partikel zu erhalten.

Eine Weiterentwicklung des erfindungsgemäßen Verfahrens besteht darin, daß der Strömungskonzentrator in einen chemischen Reaktionsprozeß integriert wird, bei dem eine Dispersion entsteht, wobei die Partikel und die Flüssigkeit während der laufenden chemischen Reaktion voneinander getrennt werden.

Eine zur Durchführung der Strömungskonzentration geeignete Vorrichtung besteht erfindungsgemäß aus einer Kapillare mit einem Durchmesser von 0,5 μm bis 10 mm, vorzugsweise 10 μm bis 10 mm, in der die aufzukonzentrierende Suspension als Hauptströmung geführt wird, an dessen Umfang in gleicher axialer Höhe gleichmäßig verteilt mehrere, vorzugsweise 3 bis 6 Bohrungen mit Durchmessern zwischen 10 % und 90 %, besonders bevorzugt zwischen 40 % und 70 % des Kanaldurchmessers angebracht sind.

Alternativ kann auch eine Vorrichtung benutzt werden, die erfindungsgemäß aus einem ebenen Kanal mit rechteckigem Querschnitt und mit einer Spaltweite von 0,5 μm bis 10 mm, vorzugsweise 10 μm bis 10 mm besteht, in dem die aufzukonzentrierende Suspension als Hauptströmung geführt wird und die an gegenüber liegenden Wänden in gleicher axialer Höhe zwei Spalte mit Spaltweiten zwischen 5 % und 50 %, bevorzugt zwischen 10 % und 30 % der Kanalweite aufweist.

Die Bohrungen der Spalte zum Abzug der an Partikeln verarmten Teilströme werden vorzugsweise so dimensioniert, daß ihre Spaltweite bzw. Durchmesser das 2- bis 10-fache, bevorzugt das 2- bis 5-fache des Medianwertes der Partikelgrößenverteilung des Aufgabestroms beträgt.

Vorteilhaft werden in Strömungsrichtung gesehen kaskadenförmig mehrere, vorzugsweise 20 bis 40 solcher Bohrungs- bzw. Spaltsysteme hintereinander geschaltet. Dabei wird der Strömungskonzentrator zweckmäßig so dimensioniert, daß der Gesamtquerschnitt der Öffnungen in gleicher axialer Höhe das 0,5- bis 4-fache, vorzugsweise das 1- bis 2-fache des Kanal- bzw. Kapillarenquerschnitts beträgt.

Im folgenden wird die Erfindung anhand von Zeichnungen und Ausführungsbeispielen näher erläutert. Es zeigen:

Figur 1      eine Konzentratorstufe mit Strömungspfeilen zur Erläuterung der physikalischen Wirkungsweise,

Figur 2      einen Querschnitt durch die Konzentratorstufe gemäß Figur 1,

Figur 3      einen Strömungskonzentrator mit mehreren kaskadenförmig hintereinander geschalteten Konzentratorstufen,

Figur 4      den Partikeldurchschlaganteil als Funktion einer normierten Partikelgröße mit experimentell bestimmten und theoretisch berechneten Kurven,

Figur 5      den Gesamtkonzentratanteil bei einem mehrstufigen Strömungskonzentrator als Funktion der Zahl der Konzentratorstufen und

Figur 6      den Konzentrationsfaktor bzw. den Partikelabscheidegrad bei der Untersuchung von kugelförmigen Partikeln in einer Flüssigkeit mit gleicher Dichte in Abhängigkeit der Stufenzahl.

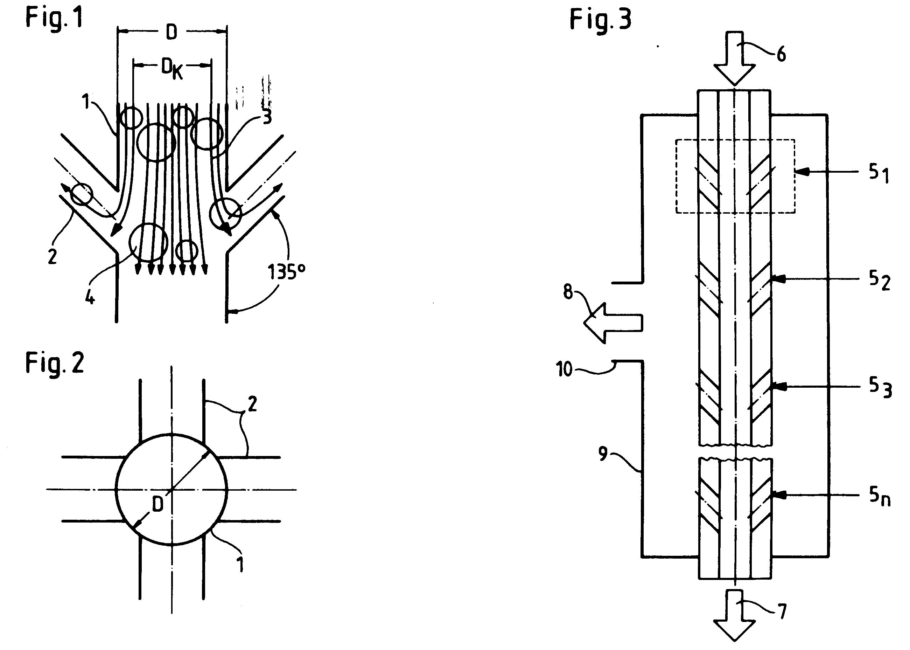

Das Trennprinzip des Strömungskonzentrators beruht auf der Ausschleusung einer partikelfreien wandnahen Zone aus einem durch einen engen Kanal fließenden Suspensionsstrom. Der Kanal kann ein Spalt mit ebenen Begrenzungsflächen oder wie in Figur 1 dargestellt, eine Kapillare 1 mit Kreisquerschnitt sein. Die Trennstufe besteht in diesem Fall aus vier gleichmäßig über den Umfang verteilten, unter einem Winkel von 135° an die Kapillare 1 angesetzten Abscheiderohren 2. Im Idealfall werden die Abscheiderohre 2 durch einen trichterförmigen Abscheidekanal ersetzt, der sich an einen Ringspalt in der Kapillare anschließt.

Der Trenneffekt beruht darauf, daß im Vergleich zu dem durch die Kapillare 1 zugeführten Aufgabestrom die Partikelkonzentration in dem durch die Kapillare 1 weiterfließenden Hauptstrom angereichert und in den durch die Abscheiderohre 2 abgezogenen Teilströmen verdünnt wird.

Wie in Figur 1 gezeigt, teilen die Grenzstromlinien 3 den geradeaus strömenden Kernstrom vom seitlich abgezogenen Grenzschichtstrom. Durch einen rein geometrischen Sperreffekt wird ausgeschlossen, daß Partikel 4, deren Radius die Dicke $x_T = \frac{1}{2}(D-D_k)$ der Grenzschicht übersteigt, in die ausgeschleuste Flüssigkeit gelangen. Dies gilt übrigens auch für verformbare Partikel, die durch Strömungskräfte in der Kernströmung gehalten werden.

Kleinere Partikel, deren Radius die Dicke der Grenzschicht unterschreitet, werden nicht mehr vollständig, sondern nur noch in einem Anteil zurückgehalten, der mit abnehmendem Verhältnis von Partikelradius zu Grenzschichtradius abnimmt.

Mit einer einzelnen Trennstufe, wie sie in Figur 1 und 2 dargestellt ist, lassen sich nur ein begrenzter Flüssigkeitsabtrenngrad und folglich ein begrenzter Konzentrationsfaktor erreichen. Für eine Ausführung mit einem Durchmesser D = 1 mm und vier im Winkel von 135° auf dem Umfang angebrachten Abscheiderohren 2 mit Bohrungen von 0,6 mm ⌀ ergab sich beispielsweise bei freiem Auslauf aus Flüssigkeits- und Konzentratöffnungen ein elementarer Flüssigkeitsabtrenngrad λ von 5 % bis 6 %. Bei vier Öffnungen von 0,6 mm ⌀ beträgt der Gesamtquerschnitt das 1,44-fache des Querschnitts der Kapillare von 1 mm ⌀. Der elementare Flüssigkeitsabtrenngrad ist definiert als das Verhältnis von Abstrom $\Delta \dot{V}$ der wandnahen partikelarmen Flüssigkeit zum Zustrom $\dot{V}$ :

$$\lambda = \frac{\Delta \dot{V}}{\dot{V}}$$

Der Zustrom $\dot{V}$ wird mit einer Pumpe eingestellt. Selbstverständlich kann man durch Androsseln des Abstroms auf der Flüssigkeitsseite bzw. auf der Konzentratseite den elementaren Flüssigkeitsabtrenngrad λ vermindern oder erhöhen.

Der elementare Konzentrationsfaktor $\kappa$ wird als Verhältnis der Partikelkonzentration $C + \Delta C$ im Konzentratablauf zur Partikelkonzentration $\Delta C$ im Zulauf definiert:

$$\kappa = \frac{C + \Delta C}{C}$$

Für technische Aufgabenstellungen sind ein elementarer Flüssigkeitsabtrenngrad von 5 % bis 6 % und ein daraus bei vollständiger Trennung folgender elementarer Konzentrationsfaktor von ca. 1,05 bis 1,06 in der Regel nicht ausreichend. Deshalb werden für die technische Anwendung mehrere Konzentratortrennstufen 5 gemäß Figur 3 kaskadenförmig hintereinandergeschaltet.

Der aus n Trennstufen bestehende Strömungskonzentrator spaltet die Aufgabesuspension 6 mit dem Durchsatz $\dot{V}_A$ und der Partikelkonzentration $C_A$ in ein Konzentrat 7 und (im Idealfall partikelfreie) Flüssigkeit 8 auf. Man kennzeichnet die Funktion des Strömungskonzentrators aus n Stufen durch den Konzentrationsfaktor $K = C_K/C_A$, der sich als n-te Potenz des elementaren Konzentrationsfaktors ergibt, und durch Konzentratanteil bzw. Flüssigkeitsabtrenngrad, die sich aus Potenzen des Komplements zum elementaren Flüssigkeitsabtrenngrad ergeben.

Man erhält insgesamt einen Konzentrationsfaktor

$$K = \kappa^n,$$

einen Konzentratanteil

$$\dot{V}_K/\dot{V}_A = (1-\lambda)^n$$

und einen Flüssigkeitsabtrenngrad

$$\dot{V}_L/\dot{V}_A = 1-(1-\lambda)^n.$$

Ein Strömungskonzentrator mit n Trennstufen $5_n$ wird durch n in Strömungsrichtung aufeinanderfolgende Bohrungssysteme realisiert. Beispielsweise wurden Ausführungen mit bis zu 75 Elementen getestet; der Rohrdurchmesser D variierte zwischen 1 und 2 mm. Damit wurden beispielsweise mit Drücken von 10 bar Durchsätze zwischen 100 und 500 l/h erzielt. Die in den einzelnen Trennstufen $5_n$ abgezogenen Teilströme werden durch den Sammelschacht 9 und den Abfluß 10 abgeleitet.

Bei der Ausführung mit einem ebenen Kanalspalt lassen sich wesentlich höhere Durchsätze erzielen. Dies gilt auch für rotationssymmetrische Ausführungen mit größerem Rohrdurchmesser D. Andererseits steht für einen bestimmten elementaren Flüssigkeitsabtrenngrad die Grenzpartikelgröße $X_T$ in einem bestimmten Verhältnis zum Rohrdurchmesser D. Bei Kleineren Verhältnissen x/D ist keine vollständige Abscheidung mehr gewährleistet.

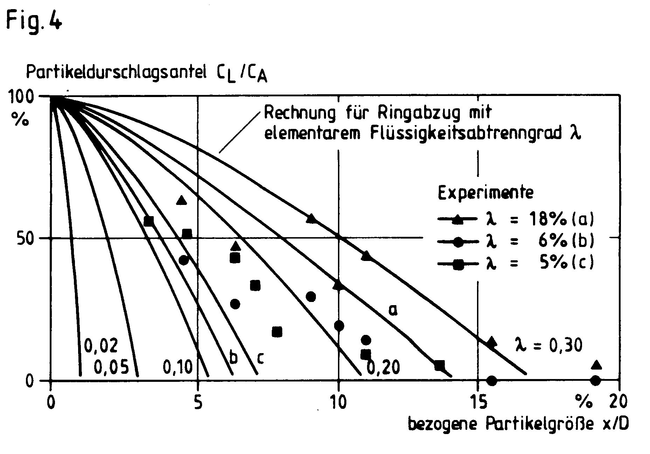

Figur 4 zeigt den Partikeldurchschlagsanteil, definiert als Konzentration $C_L$ in der ausgeschleusten Flüssigkeit bezogen auf die Konzentration $C_A$ in der Aufgabe als Funktion der bezogenen Partikelgröße X/D. Die für turbulente Strömung bei Reynoldszahlen zwischen $10^4$ und $10^5$ berechneten Kurven betreffen ideale Ringausschleusung mit elementaren Flüssigkeitsabtrenngraden $\lambda$ zwischen 2 % und 30 %. Beispielsweise erreicht man theoretisch aufgrund des Sperreffektes bei einem elementaren Flüssigkeitsabtrenngrad von 5 % eine Trennkorngröße von 3 % des Rohrdurchmessers D. Bei Unterschreiten der Trennkorngröße steigt der Partikeldurchschlagsanteil steil an. Die mit a, b und c gekennzeichneten Kurven betreffen gerechnete Werte, bei denen die nicht ideale Ausschleusung durch 4 Abscheiderohre zugrunde gelegt wurde. Bei der Darstellung der entsprechenden experimentellen Ergebnisse mit kugelförmigen Partikeln wurde der Medianwert der realen Partikelgrößenverteilung zur Definition von X/D herangezogen. Wegen des Feinanteils der Verteilung ergeben sich flacher auslaufende Kurven als berechnet.

Bei freiem Auslauf aus Flüssigkeits- und Konzentratseite liegt der elementare Flüssigkeitsabtrenngrad $\lambda$ der untersuchten Strömungskonzentratorausführungen bei etwa 5 bis 6 %. Durch Androsseln auf der Konzentratseite läßt sich $\lambda$ erhöhen.

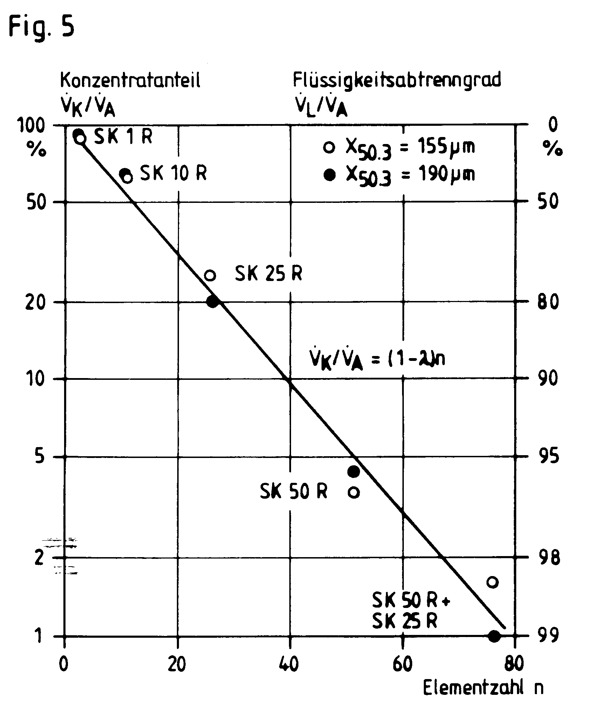

Figur 5 zeigt für den Betrieb des Strömungskonzentrators ohne Androsseln den Gesamtkonzentratanteil bzw. den Gesamtflüssigkeitsabtrenngrad als Funktion der Zahl n der Trennstufen. Die Partikeln waren kugelförmig. Ihre Dichte stimmte mit der Flüssigkeitsdichte überein. Die Medianwerte $X_{50,3}$ der beiden untersuchten Partikelgrößenverteilungen lagen bei 155 μm bzw. 190 μm. Mit n = 25 Stufen erreicht man einen Flüssigkeitsabtrenngrad von 75 %; mit 50 bzw. 75 Stufen erreicht man schon einen Flüssigkeitsabtrenngrad von 95 bzw. 99 %.

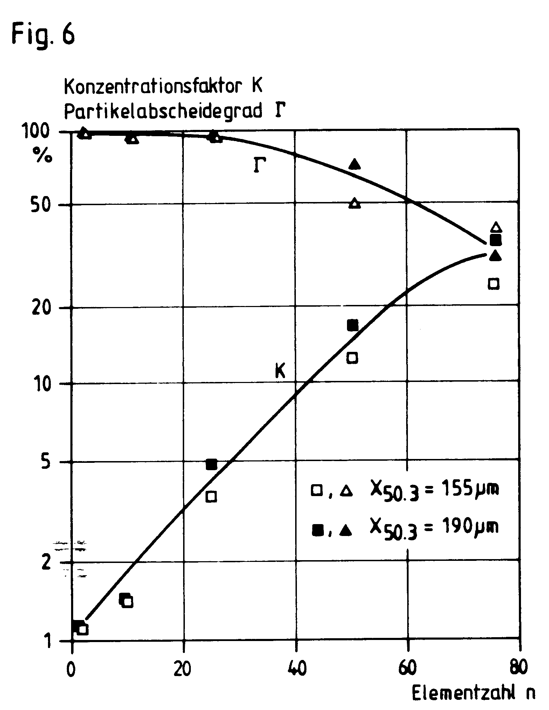

Figur 6 zeigt für dieselben Experimente mit kugelförmigen Partikeln in Flüssigkeit gleicher Dichte den erzielten Konzentrationsfaktor K. Mit n = 75 Elementen wurde eine Aufkonzentrierung um den Faktor 30 erzielt. Im dargestellten Beispiel wurde die Partikelvolumenkonzentration von 0,5 % auf 15 % erhöht. Systematische Untersuchungen mit Variation der Aufgabekonzentration haben gezeigt, daß Konzentrationsfaktor und Abscheidegrad bis zu Endvolumenkonzentrationen von 30 % konzentrationsunabhängig sind. Diese Grenze wird allein durch die eingeschränkte Fließfähigkeit hochkonzentrierter Suspensionen vorgegeben.

Allerdings reduziert sich bei hohen Flüssigkeitsabtrenngraden und entsprechend geringen Konzentratanteilen wegen der geringen Strömungsgeschwindigkeit der Hauptströmung und dem Umschlag von turbulenter zu laminarer Strömung der Partikelabscheidegrad $\Gamma$. Nahezu vollständige Partikelabscheidung wurde noch mit n = 25 Elementen und Konzentrationsfaktoren um 4 gewährleistet. Ist auch bei höheren Konzentrationsfaktoren eine nahezu vollständige Partikelabscheidung gefordert, so eignet sich die Konzentrierung in mehreren Stufen beispielsweise jeweils um den Faktor 4.

Mit dem erfindungsgemäßen Strömungskonzentrator steht somit ein einfaches System zur Aufkonzentrierung von Suspensionen zur Verfügung, das sowohl bei verschwindender Dichtedifferenz, bei der Sedimentationsverfahren versagen, als auch bei verformbaren Partikeln, wo Kuchen- oder Querstromfiltrationsverfahren versagen, angewandt werden kann.

Der Strömungskonzentrator löst das Problem der Aufkonzentrierung von Partikeln, auch wenn diese verformbar sind und keine Dichtedifferenz zur Flüssigkeit aufweisen. Die erreichbare Endkonzentration wird nur durch die Grenze der Fließfähigkeit der Suspension eingeschränkt. Die erreichbare Volumenkonzentration kann folglich, je nach Stoffsystem, bis ca. 60 % gehen. Die Aufgabekonzentration liegt bevorzugt im Bereich von 0,1 bis 10 %.

Je nach Anforderungen an den Partikelabscheidegrad ist eine einstufige Konzentrierung ausreichend oder eine mehrstufige Konzentrierung erforderlich. Bei mehrstufiger Fahrweise sind mit dem Strömungskonzentrator Partikelvolumenkonzentrationen unter 0,1 % in der auszuschleusenden Flüssigkeit zu erreichen.

Die als Beispiel mitgeteilten experimentellen Ergebnisse wurden an einigen speziellen Ausführungsformen des erfindungsgemäßen Strömungskonzentrators ermittelt.

Die Abmessungen des Hauptströmungskanals müssen den Durchmesser der größten Partikel der Dispersion sicher übertreffen. Die Abmessungen der Flüssigkeitsabzugskanäle sind, ebenso wie die Zahl der kaskadenartig hintereinandergeschalteten Elemente, dem durch die Trennaufgabe vorgegebenen Teilungsverhältnis und dem zulässigen Partikelgehalt im Flüssigkeitsablauf und dem Feinanteil der Partikelgrößenverteilung der Dispersion anzupassen. Hierzu dienen die mitgeteilten experimentellen und theoretischen Ergebnisse.

Bei der Anwendung zur Abtrennung von Mikrogelpartikeln oder makroskopischen Gelpartikeln aus Polymerlösungen mit Partikelgrößen beispielsweise von ca. 10 µm bis ca. 1 mm kommen Hauptkanalabmessungen von ca. 20 µm bis ca. 10 mm in Frage.

Bei der Anwendung in der Bioverfahrenstechnik zur Abtrennung von Mikroorganismen, z.B. Bakterien von ca. 1 µm Durchmesser, eignen sich Hauptkanalabmessungen bis herunter zu weniger als 10 µm. Für die Abtrennung von Zellbruchstücken nach einem mechanischen Zellaufschluß können Hauptkanalabmessungen bis unter 1 µm in Frage kommen. Mit den modernen Fertigungsmethoden der Mikrostrukturtechnik sind die Voraussetzungen zur Herstellung von Strömungskonzentratoren mit derart feinen Strukturen heute gegeben und auch für die technische Anwendung nutzbar.

Der Winkel der Abzugskanäle gegen die Rohrachse sollte möglichst stumpf sein, um im Falle von Partikeln mit höherer Dichte noch einen zusätzlichen Trägheitseffekt bei der Abscheidung nutzen zu können. In diesem Fall sollte der Winkel mindestens 90° betragen. Aus geometrischen und fertigungstechnischen Gründen sind Winkel über 170° problematisch.

Die Flüssigkeitsabzugskanäle können, wie in Figur 1 skizziert, als Bohrungen in ein Rohr oder einen Kanal anderer Form eingebracht sein.

Das System kann aber auch als Paket von ringförmigen Elementen zusammengesetzt sein, zwischen denen, beispielsweise durch Abstandshalter, Spalte bestehen können.

Der Durchmesser oder die Kanalweite des Hauptströmungskanals eines mehrstufigen Strömungskonzentrators müssen auch nicht konstant sein. Beispielsweise kann der Kanalquerschnitt in dem Maße stromabwärts reduziert werden, wie bereits Flüssigkeit abgezogen wurde. Durch diese Maßnahme bleibt die mittlere Strömungsgeschwindigkeit konstant. Auch müssen nicht, wie in Figur 3 bei einem vielstufigen Strömungskonzentrator, die Flüssigkeitsströme aller n Stufen vereinigt werden. Vielmehr können mehrere, maximal alle n Elemente mit getrennten Flüssigkeitsentnahmeleitungen verbunden werden. Damit wird erreicht, daß getrennte Flüssigkeitsanteile mit gegebenenfalls unterschiedlichem Partikelgehalt nicht wieder vereinigt werden. Da sich die mit den getrennten Flüssigkeitsentnahmeleitungen ausgeschleusten Partikelfraktionen auch in der mittleren Partikelgröße unterscheiden, eignet sich der Strömungskonzentrator in dieser Ausführungsform auch zum Klassieren.

Ein wichtiges Anwendungsbeispiel des Strömungskonzentrators ist die Abtrennung von Mikrogelpartikeln aus Polymerlösungen und -schmelzen. Solche Mikrogelpartikel führen zu schwerwiegenden Qualitätseinbußen, z.B. aufgrund von optischen Inhomogenitäten oder zu Störungen, z.B. zum Faserriß beim Spinnvorgang. Probleme bei der Mikrogelabscheidung ergeben sich unter anderem durch die geringe Dichtedifferenz zur umgebenden Flüssigkeit und durch die Verformbarkeit dieser Partikel.

Ähnliche Voraussetzungen - geringe Dichtedifferenz und Verformbarkeit - sind auch bei verschiedenen Aufgabenstellungen in der Bioverfahrenstechnik gegeben. Der Strömungskonzentrator eignet sich zum Aufkonzentrieren von Mikroorganismen wie Bakterien, Hefen oder Schimmelpilzen. Grundsätzlich lassen sich auch Zellbruchstucke aufkonzentrieren, wenn mit modernen Methoden der Mikrostrukturtechnik Kanäle mit Durchmessern bzw. Weiten in der Größenordnung µm gefertigt werden.

Weiterhin eignet sich der Strömungskonzentrator für die Aufkonzentrierung von Emulsionen (Öl-in-Wasser oder Wasser-in-Öl-Emulsionen) und zur Trennung von wäßrigen Zweiphasensystemen.

Der Strömungskonzentrator eignet sich nicht nur als kontinuierlicher Trennschritt im Anschluß an einen chemischen oder physikalischen Reaktionsprozeß, sondern laßt sich auch besonders leicht in Reaktionsprozesse integrieren. Damit lassen sich Reaktionen selektiver führen, wenn beispielsweise gewünschte Reaktionsprodukte aus dem Prozeß ausgeschleust werden, die zu unerwünschten Folgeprodukten weiterreagieren können.

**Patentansprüche**

1. Verfahren zur Aufkonzentrierung von suspendierten Partikeln in einer Flüssigkeit, dadurch gekennzeichnet, daß man die Suspension als Aufgabestrom (6) durch einen engen Kanal oder eine Kapillare (1) mit einer Spaltweite bzw. mit einem Durchmesser von 0,5 μm bis 10 mm, vorzugsweise 10 μm bis 10 mm, strömen läßt und am Umfang des Kanals bzw. der Kapillare (1) durch Öffnungen in der Kanalwand bzw. Kapillarenwand, deren Spaltweite bzw. Durchmesser größer ist als der Medianwert der Partikelgrößenverteilung des Aufgabestroms (6), Teilströme von der Wand aus abzieht, wobei aufgrund der im Aufgabestrom in Wandnähe abnehmenden Partikelkonzentration im Vergleich zum Aufgabestrom (6) die Partikelkonzentration im Hauptstrom angereichert und in den Teilströmen verdünnt wird.

2. Verfahren nach Anspruch 1, dadurch gekennzeichnet, daß der wandnahe Teilstrom mit einer Richtungskomponente entgegen dem Hauptstrom bevorzugt mit einem Umlenkwinkel zwischen 120° und 150° abgezogen wird.

3. Verfahren nach Anspruch 1 bis 2 dadurch gekennzeichnet, daß im Hauptstrom turbulente Strömungsbedingungen vorherrschen.

4. Verfahren nach Anspruch 1 bis 3, dadurch gekennzeichnet, daß in den Teilströmen laminare Strömungsbedingungen vorliegen.

5. Verfahren nach Anspruch 1 bis 4, dadurch gekennzeichnet, daß der Teilmengenstrom aus den Öffnungen in gleicher axialer Höhe 2 % bis 10 % vorzugsweise 4 % bis 7 % des Hauptmengenstroms beträgt.

6. Verfahren nach Anspruch 1 bis 5, dadurch gekennzeichnet, daß in Strömungsrichtung hintereinander kaskadenförmig jeweils auf gleicher axialer Höhe Teilströme abgezogen werden.

7. Verfahren nach Anspruch 6, dadurch gekennzeichnet, daß die aus verschiedenen axialen Höhen abgezogenen Teilströme getrennt abgeführt werden, um Teilströme unterschiedlicher Partikelkonzentration und unterschiedlicher Größenverteilung der Partikeln zu erhalten.

8. Verfahren nach Anspruch 6 bis 7, dadurch gekennzeichnet, daß die Summe aller Teilströme bis zu 99 % des Aufgabestroms (6) beträgt.

9. Verfahren nach Anspruch 1 bis 8, dadurch gekennzeichnet, daß Partikel und Flüssigkeit während einer laufenden chemischen Reaktion voneinander getrennt werden.

10. Vorrichtung zur Aufkonzentrierung von suspendierten Partikeln in einer Flüssigkeit, nach dem Verfahren gemäß den Ansprüchen 1 bis 5, dadurch gekennzeichnet, daß an einer Kapillare (1) mit einem Durchmesser von 0,5 μm bis 10 mm, vorzugsweise 10 μm bis 10 mm, in der die aufzukonzentrierende Suspension (6) als Hauptströmung geführt wird, auf dem Umfang mehrere, vorzugsweise 3 bis 6 Bohrungen mit Durchmessern zwischen 10 % und 90 %, bevorzugt zwischen 40 % und 70 % des Kapillarendurchmessers in gleicher axialer Höhe zum Abzug von Teilströmen angebracht sind.

11. Vorrichtung zur Aufkonzentrierung von suspendierten Partikeln in einer Flüssigkeit, nach dem Verfahren gemäß den Ansprüchen 1 bis 5, dadurch gekennzeichnet, daß an einem ebenen Kanal mit rechteckigem Querschnitt mit einer Spaltweite von 0,5 μm bis 10 mm, vorzugsweise 10 μm bis 10 mm, in dem die aufzukonzentrierende Suspension als Hauptströmung geführt wird, an gegenüber liegenden Wänden in gleicher axialer Höhe zwei Spalte mit Spaltweiten zwischen 5 % und 50 % bevorzugt zwischen 10 % und 30 % der Kanalweite zum Abzug von Teilströmen angebracht sind.

12. Vorrichtung nach Anspruch 10 oder 11, dadurch gekennzeichnet, daß die Bohrungen oder Spalte zum Abzug der an Partikeln verarmten Teilströme einen Durchmesser bzw. eine Spaltweite vom 2- bis 10-fachen, bevorzugt vom 2- bis 5-fachen des Medianwertes der Partikelgrößenverteilung des Aufgabestroms (6) haben.

13. Vorrichtung gemäß Anspruch 10 bis 12 zur Durchführung des Verfahrens nach Anspruch 6 bis 8, dadurch gekennzeichnet, daß in Strömungsrichtung kaskadenförmig hintereinander mehrere, vorzugsweise 20 bis 40 solcher Bohrungs- bzw. Spaltsysteme (5n) angeordnet sind.

14. Vorrichtung nach Anspruch 10 bis 12, dadurch gekennzeichnet, daß der Gesamtquerschnitt der Öffnungen in gleicher axialer Höhe das 0,5- bis 4-fache, vorzugsweise das 1- bis 2-fache des Kanal- bzw. Kapillarenquerschnitts beträgt.

## Claims

1. Process for concentrating suspended particles in a liquid, characterised in that the suspension is caused to flow as a feed scream (6) through a narrow channel or a capillary (1) having a gap width or a diameter of 0.5 μm to 10 mm, preferably 10 μm to 10 mm, and at the circumference of the channel or capillary (1) partial streams are abstracted from the channel wall or capillary wall through openings in the latter whose gap width or diameter is greater than the median value of the particle size distribution of the feed stream (6), the particle concentration in the main stream being enriched compared to the feed stream (6) and in the partial streams being depleted compared to the feed stream (6) on account of the decreasing particle concentration in the feed stream (6) in the vicinity of the wall.

2. Process according to claim 1, characterised in that the partial stream in the vicinity of the wall having a direction component opposite to the main stream is abstracted preferably at a deflection angle of between 120° and 150°.

3. Process according to claim 1 or 2, characterised in that turbulent flow conditions prevail in the main stream.

4. Process according to claims 1 to 3, characterised in that laminar flow conditions exist in the partial streams.

5. Process according to claims 1 to 4, characterised in that the partial stream abstracted from the openings of the same axial height comprises 2% to 10%, preferably 4% to 7%, of the main stream.

6. Process according to claims 1 to 5, characterised in that partial streams are abstracted in succession in a cascade-like manner in the flow direction, and in each case at the same axial height.

7. Process according to claim 6, characterised in that the partial streams abstracted from different axial heights are removed separately in order to obtain partial streams of different particle concentration and different size distribution of the particles.

8. Process according to claim 6 or 7, characterised in that the sum of all partial streams comprises up to 99% of the feed stream (6).

9. Process according to claims 1 to 8, characterised in that particles and liquid are separated from one another during an ongoing chemical reaction.

10. Apparatus for concentrating suspended particles in a liquid, according to the process according to claims 1 to 5, characterised in that in a capillary (1) having a diameter of 0.5 μm to 10 mm, preferably 10 μm to 10 mm, through which the suspension (6) to be concentrated is fed as a main stream, a plurality, preferably 3 to 6 bores having diameters of between 10% and 90%, preferably between 40% and 70% of the capillary diameter, are arranged at the same axial height on the circumference in order to abstract partial streams.

11. Apparatus for concentrating suspended particles in a liquid, according to the process according to claims 1 to 5, characterised in that in a plane channel of rectangular cross-section having a gap width of 0.5 μm to 10 mm, preferably 10 μm to 10 mm, through which the suspension to be concentrated is fed as a main stream, two gaps having gap widths of between 5% and 50%, preferably between 10% and 30% of the channel width, are provided at the same axial height on opposite walls in order to abstract partial streams.

12. Apparatus according to claim 10 or 11, characterised in that the bores or gaps for abstracting particle-depleted partial streams have a diameter or a gap width of 2 to 10 times, preferably 2 to 5 times, the median value of the particle size distribution of the feed stream (6).

13. Apparatus according to claims 10 to 12 for carrying out the process according to claims 6 to 8, characterised in that a plurality, preferably 20 to 40 such bore or gap systems (5n) are arranged in succession in a cascade-like manner in the flow direction.

14. Apparatus according to claims 10 to 12, characterised in that the total cross-section of the openings at the same axial height is 0.5 to 4 times, preferably 1 to 2 times that of the channel or capillary cross-section.

## Revendications

1. Procédé pour augmenter la concentration de particules en suspension dans un liquide, caractérisé en ce qu'on laisse s'écouler la suspension sous forme de courant d'alimentation (6) à travers un canal étroit ou un capillaire (1) possédant une largeur de fente, respectivement un diamètre de 0,5 μm à 10 mm, de préférence de 10 μm à 10 mm et, à la périphérie du canal, respectivement du capillaire (1), on extrait des courants partiels à partir de la paroi via des ouvertures pratiquées dans la paroi du canal, respectivement dans la paroi du capillaire dont la largeur de fente, respectivement le diamètre est supérieur à la valeur médiane de la distribution granulométrique du courant d'alimentation (6), dans lequel, sur base de la concentration décroissante des particules dans le courant d'alimentation à proximité de la paroi, on enrichit la concentration des particules dans le courant principal par rapport à celle du courant d'alimentation (6) et on la réduit dans les courants partiels.

2. Procédé selon la revendication 1, caractérisé en ce qu'on extrait le courant partiel proche de la paroi en respectant un composant d'orientation par rapport au courant principal de préférence en respectant un angle de déviation entre 120° et 150°.

3. Procédé selon les revendications 1 à 2, caractérisé en ce que des conditions d'écoulement turbulent prédominent dans le courant principal.

4. Procédé selon les revendications 1 à 3, caractérisé en ce que des conditions d'écoulement laminaire règnent dans les courants partiels.

5. Procédé selon les revendications 1 à 4, caractérisé en ce que le courant de quantité partielle provenant des ouvertures à la même hauteur axiale représente de 2% à 10%, de préférence de 4% à 7% du courant de quantité principale.

6. Procédé selon les revendications 1 à 5, caractérisé en ce que, dans la direction d'écoulement, on extrait des courants partiels successifs en cascade, respectivement à la même hauteur axiale.

7. Procédé selon la revendication 6, caractérisé en ce qu'on évacue séparément les courants partiels extraits à différentes hauteurs axiales pour obtenir des courants partiels possédant des concentrations de particules différentes et des distributions granulométriques différentes des particules.

8. Procédé selon les revendications 6 à 7, caractérisé en ce que la somme de tous les courants partiels représente jusqu'à 99% du courant d'alimentation (6).

9. Procédé selon les revendications 1 à 8, caractérisé en ce qu'on sépare l'un de l'autre les particules et le liquide au cours d'une réaction chimique en continu.

10. Dispositif pour augmenter la concentration de particules en suspension dans un liquide conformément au procédé selon les revendications 1 à 5, caractérisé en ce qu'on applique à la périphérie d'un capillaire (1) possédant un diamètre de 0,5 $\mu$m à 10 mm, de préférence de 10 $\mu$m à 10 mm, dans lequel on guide, sous forme de courant principal, la suspension (6) dont on doit augmenter la concentration, plusieurs, de préférence de 3 à 6 alésages possédant des diamètres représentant entre 10% et 90%, de préférence entre 40% et 70% du diamètre du capillaire, à la même hauteur axiale, pour l'extraction de courants partiels.

11. Dispositif pour augmenter la concentration de particules en suspension dans un liquide conformément au procédé selon les revendications 1 à 5, caractérisé en ce qu'on applique sur les parois opposées d'un canal plan à section transversale rectangulaire, possédant une largeur de fente de 0,5 $\mu$m à 10 mm, de préférence de 10 $\mu$m à 10 mm, dans lequel on guide, sous forme de courant principal, la suspension dont on doit augmenter la concentration, à la même hauteur axiale, deux fentes possédant des largeurs de fente représentant entre 5% et 50%, de préférence entre 10% et 30% de la largeur du canal, pour l'extraction de courants partiels.

12. Dispositif selon la revendication 10 ou 11, caractérisé en ce que les alésages ou les fentes pour l'extraction des courants partiels appauvris en particules possèdent un diamètre, respectivement une largeur de fente représentant de 2 à 10 fois, de préférence de 2 à 5 fois la valeur médiane de la distribution granulométrique du courant d'alimentation (6).

13. Dispositif selon les revendications 10 à 12, pour la mise en oeuvre du procédé selon les revendications 6 à 8, caractérisé en ce que, dans la direction d'écoulement, on dispose plusieurs, de préférence de 20 à 40, systèmes d'alésages, respectivement de fentes (5n) montés l'un derrière l'autre en cascade.

14. Dispositif selon les revendications 10 à 12, caractérisé en ce que la section transversale totale des ouvertures à la même hauteur axiale représente de 0,5 à 4 fois, de préférence de 1 à 2 fois la section transversale du canal, respectivement du capillaire.

Fig. 1

Fig. 2

Fig. 3

EP 0 641 587 B1

Fig. 4

Partikeldurschlagsantel $C_L / C_A$

Rechnung für Ringabzug mit
elementarem Flüssigkeitsabtrenngrad $\lambda$

Experimente
$\lambda = 18\%$ (a)
$\lambda = 6\%$ (b)
$\lambda = 5\%$ (c)

0,02
0,05
0,10
b
c
0,20
a
$\lambda = 0,30$

bezogene Partikelgröße x/D

## Fig. 5

Konzentratanteil $\dot{V}_K/\dot{V}_A$      Flüssigkeitsabtrenngrad $\dot{V}_L/\dot{V}_A$

- SK 1 R
- SK 10 R
- SK 25 R
- SK 50 R
- SK 50 R + SK 25 R

$X_{50.3} = 155\,\mu m$
$X_{50.3} = 190\,\mu m$

$$\dot{V}_K/\dot{V}_A = (1-\lambda)n$$

Elementzahl n

# Fig. 6